(19) **Europäisches Patentamt European Patent Office Office européen des brevets**

(11) **EP 1 502 978 B1**

(12) **EUROPEAN PATENT SPECIFICATION**

(45) Date of publication and mention
of the grant of the patent:
**09.09.2009 Bulletin 2009/37**

(51) Int Cl.:
*D03D 49/10* (2006.01)          *B65H 63/08* (2006.01)
*G01B 21/10* (2006.01)          *G01N 33/36* (2006.01)

(21) Application number: **04016603.5**

(22) Date of filing: **14.07.2004**

(54) **Method and apparatus for calculating winding diameter in textile machine**

Verfahren und Vorrichtung zum Berechnen des Wicklungsdurchmessers in einer Textilmaschine

Procédé et dispositif de calcul du diamètre d'enroulement dans une machine textile

(84) Designated Contracting States:
**BE DE IT**

(30) Priority: **01.08.2003   JP 2003285071**
**16.03.2004   JP 2004075218**

(43) Date of publication of application:
**02.02.2005   Bulletin 2005/05**

(60) Divisional application:
**07001656.3 / 1 775 360**

(73) Proprietor: **TSUDAKOMA KOGYO KABUSHIKI KAISHA**
**Kanazawa-shi,**
**Ishikawa-ken 921-8650 (JP)**

(72) Inventors:
 • **Yamamoto, Akihiko**
   **Kanazawa-shi**
   **Ishikawa-ken 921-8650 (JP)**
 • **Ishita, Tomokazu**
   **Kanazawa-shi**
   **Ishikawa-ken 921-8650 (JP)**
 • **Matsuyama, Yutaka**
   **Kanazawa-shi**
   **Ishikawa-ken 921-8650 (JP)**

(74) Representative: **Müller-Boré & Partner**
**Patentanwälte**
**Grafinger Strasse 2**
**81671 München (DE)**

(56) References cited:
**EP-A- 0 306 706          EP-A- 0 335 080**
**EP-A- 1 277 682          DE-A1- 4 304 955**
**US-A- 3 858 415          US-A- 4 426 856**
**US-B1- 6 292 989**

**Description**

BACKGROUND OF THE INVENTION

The present invention relates to a method and apparatus for calculating the diameter of a winding in a textile machine such as a weaving machine or a preparatory machine.

Sheet-like matter including a warp sheet consisting of a plurality of warp threads arranged like a sheet is wound around a winding core to constitute a winding. The winding core is rotatably mounted in a textile machine.

If the sheet-like matter is a warp sheet in a weaving machine or woven cloth in a sewing machine, the winding core is rotated at a constant rotational angular speed to unwind and feed out the winding. The sheet-like matter fed out is fed at a given speed into a sheet-like matter-handling machine such as a weaving machine in weaving machinery or a cutter in a sewing machine placed downstream.

The diametrical dimension of the winding decreases as the amount of the sheet-like matter pulled out increases. However, the rotational angular speed of the winding core is set constant. Therefore, the speed at which the unwound sheet-like matter is pulled out decreases gradually. This imparts tensile force on the sheet-like matter between the sheet-like matter-handling machine and the winding.

To prevent such tensile force from being applied to the sheet-like matter, a method consisting of calculating the diameter of the winding and controlling the rotational angular speed of the winding core according to the calculated winding diameter is adopted.

One known method of calculating the winding diameter consists of detecting the distance between the warp thread wound on the top beam and a non-contact sensor by a non-contact sensor and calculating the diameter of the warp thread wound on the top beam, as shown in JP-A-04-289242.

However, the non-contact sensor is expensive. Atextile machine fitted with such non-contact sensors is also expensive. Furthermore, the thicknesswise dimension of the sheet-like matter including the warp sheet that is to be measured by the non-contact sensors is smaller than the dimensional accuracy at which the non-contact sensors can measure. Consequently, measuring error is produced.

EP 1 277 682 discloses a winder comprising a winding body for winding the material, a drive device for driving this winding body and a device for controlling the winder, which is provided for automatically controlling the winding torque of the winder as a function of the desired tractive force in the material.

**SUMMARY OF THE INVENTION**

It is an object of the present invention to accurately calculate a winding diameter close to an actually measured value without using sensors.

In order to achieve the above object, in a first aspect, the present invention provides a method according to claim 1.

Furthermore, in a second aspect, the present invention provides an apparatus according to claim 10.

BRIEF DESCRIPTION OF THE DRAWINGS

Fig. 1 is a block diagram of a weaving machine using a method of calculating a winding diameter in accordance with the present invention, the method being utilized in textile machines.
Fig. 2 is a figure illustrating various dimensions of a winding core and sheet-like matter for illustrating a method of calculating the winding diameter in accordance with a first embodiment of the invention.
Fig. 3 is a graph in which diametrical dimension *D(i)* of the winding is plotted against the number of woven sheets, the graph being obtained by a method of calculating the winding diameter in accordance with a second embodiment of the invention.

DESCRIPTION OF THE PREFERRED EMBODIMENTS

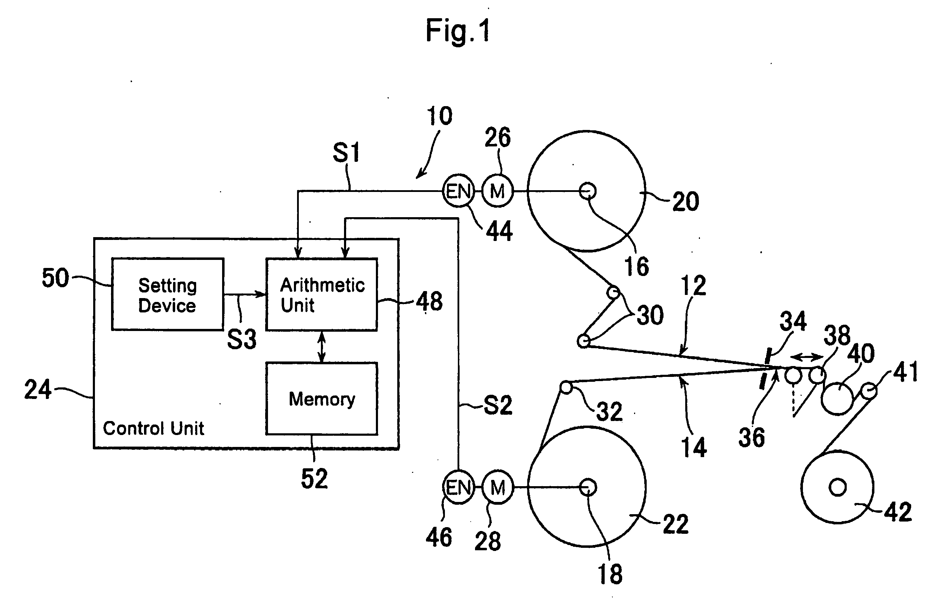

Referring to Fig. 1, a pile fabricmachine 10 is described as an example of textile machine using a method of calculating a winding diameter.

The pile fabric machine 10 is fitted with a pile beam 20 and a groundbeam 22 having winding cores 16 and 18, respectively, on which warp sheets 12 and 14 are respectively wound. In each of the warp sheets 12 and 14, a plurality of warp threads are arranged like a sheet.

The pile beam 20 and ground beam 22 are connected to the output shafts of motors 26 and 28, respectively.

The motors 26 and 28 are rotated by drive signals output from a control unit 24 and thus controlled by the drive signals. Consequently, the motors 26 and 28 are rotated in given directions.

The warp sheets 12 and 14 pulled out from the pile beam 20 and ground beam 22, respectively, are caught on a pile tension roll 30 and a ground tension roll 32, respectively, and guided to a reed 34. The warp sheets 12 and 14 guided to the reed 34 are beaten together with weft thread to create woven cloth 36. The woven cloth 36 is taken up on a cloth roller 42 via a terry motion mechanism 38, a friction roll 40, and a guide roll 41.

The output shafts of the motors 26 and 28 are mounted to shafts of encoders 44 and 46 so as to be non-rotatable relative to each other, the encoders acting as rotational angle detectors. The shafts of the encoders are used for measurement.

The encoders 44 and 46 detect the rotational angles of the output shafts of the motors 26 and 28, respectively, and output rotational angle signals S1 and S2 to an arithmetic unit 48 in the control unit 24. In particular, each rotational angle is given by (the number of pulses obtained by counting pulse signals from the encoder 44 or 46)/(the number of pulses required to rotate the pile beam 20 or ground beam 22 once).

The arithmetic unit 48 calculates a winding diameter (described later) based on values stored in a memory 52, such as the thicknesswise and lengthwise dimensions of the warp sheets 12 and 14 preset by a setting device 50. Furthermore, the arithmetic unit 48 temporarily stores the calculated winding diameter and other calculated values in the memory 52 and reads in values temporarily stored in the memory 52. Accordingly, the control unit 24 and memory 52 act as a unit for calculating the winding diameter and a storage portion, respectively, in the textile machine.

[EMBODIMENT 1]

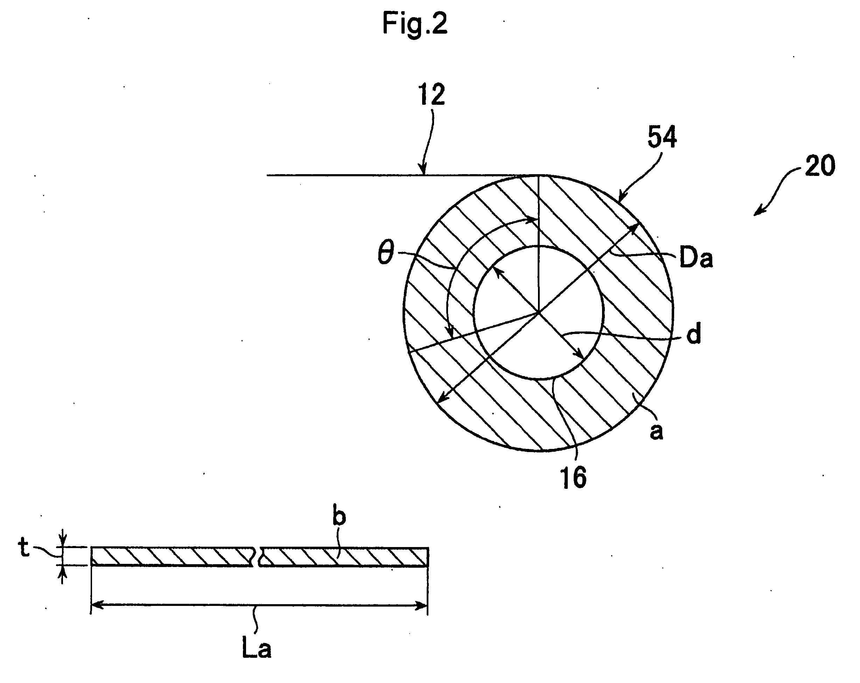

A method of calculating the diametrical dimension $Da$ of the winding on the pile beam 20 is described with reference to Fig. 2. Similar processing is performed regarding the ground beam 22.

A wound portion 54 is a range in which the warp sheet 12 is wound around a winding core 16. The total lengthwise dimension $Lmax$, i.e., the initial lengthwise dimension, of the warp sheet 12 corresponding to the wound portion 54, the total winding diametrical dimension $Dmax$, i.e., the initial winding diametrical dimension, of the warp sheet 12 of the whole wound portion, and the diametrical dimension d of the winding core 16 are previously measured.

The initial lengthwise dimension $Lmax$ of the warp sheet 12 forming the wound portion 54 and the initial winding diametrical dimension Dmax can be measured using a well-known measuring method during beam warping such as before or when the warp sheet 12 is wound on the winding core 16.

The diametrical dimension d of the winding core 16 can be measured with a measuring instrument such as a caliper before the warp sheet 12 is wound.

The previously measured initial lengthwise dimension $Lmax$ of the warp sheet 12, the initial winding diametrical dimension $D$max, and the diametrical dimension d are entered into the setting device 50 by an input device such as a touch panel mounted on the control unit 24 and stored in the memory 52.

The thicknesswise dimension $t$ of the warp sheet 12 can be previously calculated based on the cross-sectional area a (hereinafter may be referred to as the residual cross-sectional area) of the warp sheet 12 of the whole wound portion and on the initial lengthwise dimension $Lmax$ of the warp sheet 12, the whole wound portion being in the state where all the warp sheet 12 is wound around the winding core 16. The cross-sectional area a is taken within a virtual plane perpendicular to the axis of the winding core 16. Therefore, the arithmetic unit 48 calculates the value of the thicknesswise dimension t using Eq. (1) from the initial lengthwise dimension $Lmax$, initial winding diametrical dimension $Dmax$, and diametrical dimension $d$.

In particular, the cross-sectional area a is calculated from the previously measured initial winding diametrical dimension $Dmax$ of the warp sheet 12 of the whole wound portion. The thicknesswise dimension $t$ can be calculated using Eq. (1).

$$t = \frac{\pi \times (D\max \times D\max - d \times d)}{4 \times L\max} \tag{1}$$

With respect to the formula for finding the diametrical dimension of the wound portion, i.e., the diametrical dimension $Da$, the cross-sectional area (doughnut-shaped range a indicated by the hatching) of the wound portion within the virtual plane perpendicular to the axis of the winding core 16 is equal to the product of the lengthwise dimension of the warp sheet 12 (i.e., the lengthwise dimension La of the wound portion) and the thicknesswise dimension $t$ (rectangular range $b$ indicated by the hatching). Therefore, Eq. (2) is derived.

$$\frac{(Da \times Da - d \times d)\pi}{4} = t \times La \qquad (2)$$

Eq. (2) can be modified as given by Eq. (3).

$$Da = \sqrt{\frac{4 \times t \times La}{\pi} + d \times d} \qquad (3)$$

Since the warp sheet 12 is pulled out a distance equal to the weft insert pitch during one insertion of weft and moved toward the cloth roller 42, the lengthwise dimension $La$ of the wound portion can be obtained by subtracting the distance traveled (drawn-out length) from the initial lengthwise dimension $Lmax$ of the warp sheet 12. Therefore, according to Eq. (3), the winding diametrical dimension $Da$ can be obtained at high accuracy.

[EMBODIMENT 2]

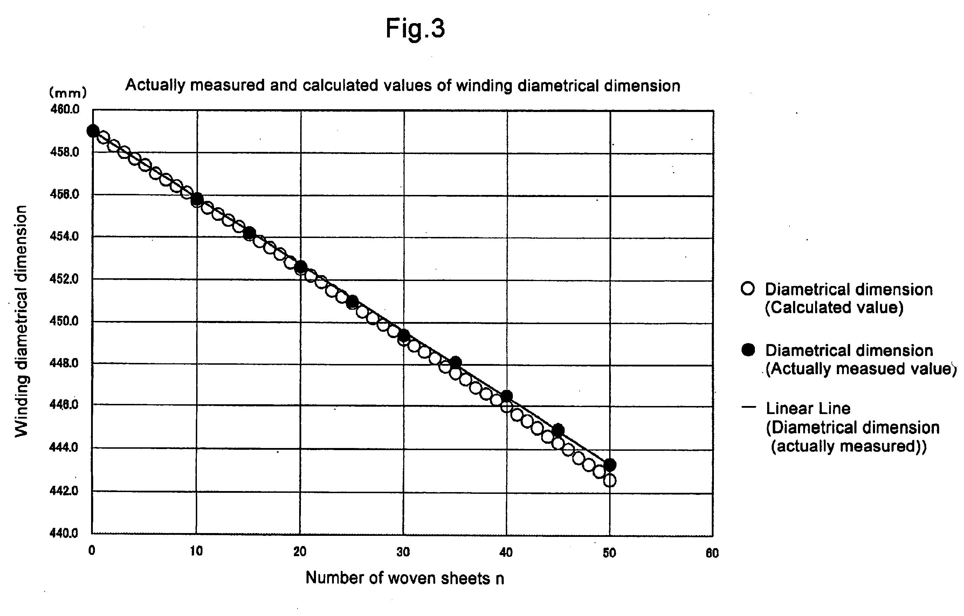

Amethod of calculating the winding diametrical dimension Da of the pile beam 20 during actual pile weaving is described with reference to Fig. 3 and Tables 1 and 2.

In Embodiment 2, the winding diametrical dimension $Da$ of the pile beam 20 is calculated using the residual thread lengthwise dimension.

First, the initial lengthwise dimension $Lmax$ of the warp sheet 12, the diametrical dimension d of the winding core 16, and the initial winding diametrical dimension $Dmax$ are actually measured. The actually measured dimensions are listed in Table 1.

Table 1

| Initial diametrical dimension of winding | Thicknesswise dimension of warp sheet | Initial lengthwise dimension | Winding core |
|---|---|---|---|
| *Dmax* | *t* | *Lmax* | *d* |
| 459.0 (mm) | 0.09466 (mm) | 1368235 (mm) | 214 (mm) |

The thicknesswise dimension t of the warp sheet 12 when the warp sheet 12 is wound around the winding core 16 is calculated, using the above Eq. (1). The calculated thicknesswise dimension $t$ is shown in Table 1.

With respect to the thicknesswise dimension $t$, the value of the thicknesswise dimension managed with a warp beam number, for example, may be directly entered.

When the whole warp sheet 12 is wound around the winding core 16, the pre-rotational diametrical dimension of the wound portion, i.e., the initial diametrical dimension of the winding, is set to $Dmax$ and $D(0)$.

(Step 1) When weaving is started, the pile beam 20 in the state where the whole warp sheet 12 is wound around the winding core 16, i.e., the winding diametrical dimension $D(i)$ is equal to the initial diametrical dimension $Dmax$ of the winding, is rotated through a given rotational angle $\theta(i)$. With respect to the rotational angle $\theta(i)$, the number of woven sheets n shown in Table 2 is set to give a weavable rotational angle. In the present embodiment, the number of picks necessary to weave one sheet of cloth for testing is 840.

Table 2

| Number of woven sheets | Drawn-out lengthwise dimension (calculated value) | Residual thread lengthwise dimension | Winding diametrical dimension (calculated value) | Winding diametrical dimension (actually measured value) | Difference |
|---|---|---|---|---|---|
| *n* | *Lb(i)* (mm) | *La(i)* (mm) | *D(i)* (mm) | (mm) | (mm) |
| 0 | | 1368235 | 459.0 | 459.0 | 0.0 |

(continued)

| Number of woven sheets | Drawn-out lengthwise dimension (calculated value) | Residual thread lengthwise dimension | Winding diametrical dimension (calculated value) | Winding diametrical dimension (actually measured value) | Difference |
|---|---|---|---|---|---|
| $n$ | $Lb(i)$ (mm) | $La(i)$ (mm) | $D(i)$ (mm) | (mm) | (mm) |
| 1 | 2496 | 1365740 | 458.7 | | |
| 2 | 2485 | 1363255 | 458.3 | | |
| 3 | 2479 | 1360777 | 458.0 | | |
| 4 | 2470 | 1358307 | 457.7 | | |
| 5 | 2477 | 1355830 | 457.4 | 457.0 | 0.4 |
| 6 | 2464 | 1353366 | 457.0 | | |
| 7 | 2453 | 1350913 | 456.7 | | |
| 8 | 2459 | 1348454 | 456.4 | | |
| 9 | 2458 | 1345995 | 456.1 | | |
| 10 | 2449 | 1343547 | 455.7 | 455.8 | -0.1 |
| 11 | 2446 | 1341100 | 455.4 | | |
| 12 | 2455 | 1338646 | 455.1 | | |
| 13 | 2446 | 1336200 | 454.8 | | |
| 14 | 2434 | 1333766 | 454.5 | | |
| 15 | 2442 | 1331324 | 454.1 | 454.2 | -0.1 |
| 16 | 2455 | 1328869 | 453.8 | | |
| 17 | 2448 | 1326421 | 453.5 | | |
| 18 | 2440 | 1323980 | 453.2 | | |
| 19 | 2443 | 1321537 | 452.8 | | |
| 20 | 2453 | 1319085 | 452.5 | 452.6 | -0.1 |
| 21 | 2449 | 1316636 | 452.2 | | |
| 22 | 2436 | 1314200 | 451.9 | | |
| 23 | 2442 | 1311758 | 451.5 | | |
| 24 | 2449 | 1309309 | 451.2 | | |
| 25 | 2442 | 1306867 | 450.9 | 451.0 | -0.1 |
| 26 | 2452 | 1304415 | 450.5 | | |
| 27 | 2444 | 1301971 | 450.2 | | |
| 28 | 2446 | 1299524 | 449.9 | | |
| 29 | 2439 | 1297085 | 449.6 | | |
| 30 | 2437 | 1294648 | 449.2 | 449.4 | -0.2 |
| 31 | 2447 | 1292201 | 448.9 | | |
| 32 | 2433 | 1289768 | 448.6 | | |
| 33 | 2431 | 1287337 | 448.3 | | |
| 34 | 2437 | 1284900 | 447.9 | | |

(continued)

| Number of woven sheets | Drawn-out lengthwise dimension (calculated value) | Residual thread lengthwise dimension | Winding diametrical dimension (calculated value) | Winding diametrical dimension (actually measured value) | Difference |
|---|---|---|---|---|---|
| n | Lb(i) (mm) | La(i) (mm) | D(i) (mm) | (mm) | (mm) |
| 35 | 2439 | 1282461 | 447.6 | 448.1 | -0.5 |
| 36 | 2444 | 1280017 | 447.3 | | |
| 37 | 2431 | 1277586 | 446.9 | | |
| 38 | 2449 | 1275137 | 446.6 | | |
| 39 | 2440 | 1272697 | 446.3 | | |
| 40 | 2429 | 1270268 | 446.0 | 446.5 | -0.5 |
| 41 | 2437 | 1267830 | 445.6 | | |
| 42 | 2435 | 1265395 | 445.3 | | |
| 43 | 2446 | 1262949 | 445.0 | | |
| 44 | 2443 | 1260506 | 444.6 | | |
| 45 | 2429 | 1258077 | 444.3 | 444.9 | -0.6 |
| 46 | 2439 | 1255638 | 444.0 | | |
| 47 | 2455 | 1253182 | 443.6 | | |
| 48 | 2455 | 1250728 | 443.3 | | |
| 49 | 2443 | 1248285 | 443.0 | | |
| 50 | 2441 | 1245844 | 442.6 | 443.3 | -0.7 |

In Table 2, the number of woven sheets $n$ is obtained by accumulatively counting woven sheets. That the number of woven sheets $n$ is zero means that the warp sheet 12 has fully been wound around the winding core 16, i.e., weaving is not yet performed.

The rotational angle θ$(i)$ indicates the rotational angle of the winding core 16 capable of weaving n (shown in Table 2) sheets. i is a number indicating the number of executions of a loop for calculating the diameter of the winding.

Furthermore, the rotational angle θ$(i)$ maybe calculated from a given time of rotation, from the tissue of the warp sheet 12 or woven cloth 36, or from the number of woven cloth sheets.

(Step 2) Then, the drawn-out lengthwise dimension of the warp sheet 12 drawn out (payed out) from the winding core 16 when the winding core 16 is rotated through the rotational angle θ$(1)$ is calculated based on the first pre-rotational diametrical dimension of the winding (i.e., initial diametrical dimension $D(0)$ of the winding) and on the rotational angle θ$(1)$.

The drawn-out lengthwise dimension can be computed using Eq. (4') obtained by substituting $i = 1$ into Eq. (4). In Eq. (4), $i$ is a number indicating the ith calculation loop, $Lb(i)$ is the $i$th drawn-out lengthwise dimension, $D(i - 1)$ is the diametrical dimension of the winding obtained after the $(i - 1)$th rotation and prior to the ith rotation, $D(0)$ is the initial diametrical dimension of the whole wound portion, i.e., the warp sheet 12 is fully wound around the winding core 16 within a virtual plane perpendicular to the axis of the winding core 16, and θ$(i)$ is the ith rotational angle in unit of °. The dimension $D(0)$ is set equal to the maximum diametrical dimension $Dmax$ of the winding.

$$Lb(i) = \frac{\pi \times D(i-1) \times \theta(i)}{360} \qquad (4)$$

$$Lb(1) = \frac{\pi \times D\max \times \theta(1)}{360} \qquad (4')$$

The first drawn-out lengthwise dimension $Lb(1)$ is calculated from a post-rotational diametrical dimension $D(0)$ of the wound portion, i.e., the maximum diametrical dimension $Dmax$ of the winding, and from the rotational angle $\theta(1)$.

(Step 3) Then, the residual thread lengthwise dimension is calculated based on the total lengthwise dimension of the warp sheet 12 and on the drawn-out lengthwise dimension.

The residual thread lengthwise dimension can be calculated using Eq. (5). $La(i)$ indicates the ith residual thread lengthwise dimension.

$$La(i) = L\max - \{Lb(i) + Lb(i-1) + \cdots + Lb(1)\} \qquad (5)$$

The residual thread lengthwise dimension $La(i)$ found using Eq. (5) is shown in the column "residual thread lengthwise dimension $La(i)$" of Table 2. The drawn-out lengthwise dimension $Lb$ (i) is also shown in the column "drawn-out lengthwise dimension $Lb(i)$" of Table 2.

(Step 4) Then, the post-rotational diametrical dimension of the wound portion (i.e., the diametrical dimension of the warp sheet 12 in the state where the warp sheet 12 is wound around the winding core 16 after the winding core 16 is rotated through a rotational angle) is calculated based on the residual thread lengthwise dimension, on the thicknesswise dimension of the wound portion (i.e., the thicknesswise dimension of the warp sheet 12 in the state where the sheet is wound around the winding core 16), and on the diametrical dimension of the winding core portion (i.e., the diametrical dimension of the winding core 16), using Eq. (6). $D(i)$ indicates the diametrical dimension after the ith rotation, $t$ indicates the thicknesswise dimension of the wound portion, and $La(i)$ indicates the residual thread lengthwise dimension.

$$D(i) = \sqrt{\frac{4 \times t \times La(i)}{\pi} + d \times d} \qquad (6)$$

(Step 5) Then, the winding diametrical dimension $D(i+1)$ obtained after the winding core 16 is rotated through the rotational angle $\theta(i+1)$ is calculated, based on the ith post-rotational winding diametrical dimension $D(i)$ obtained using Eq. (6), and by the use of the calculational method illustrated by Eqs. (4) to (6). In particular, the value of $D(i)$ is substituted into $D(i-1)$ of Eq. (4).

The ith winding diametrical dimension $D(i)$ can be calculated by repeating the calculation loop of the above-described Eqs. (4) to (6) a given number of times (50 times in Table 2). The calculation of the drawn-out lengthwise dimension $Lb(i)$ from the winding diametrical dimension $D(i)$ calculated in this way can be carried out by a well-known calculational method.

Fig. 3 is a graph showing the relation between the number of woven sheets $n$ and the winding diametrical dimension $D(i)$, the number $n$ assuming values shown in Table 2.

The values of "diametrical dimension (actually measured value) of the winding" measured every 5 woven cloth sheets are shown in Table 2. Also, the values of the "difference" between the "diametrical dimension (measured value) of the winding" and the "diametrical dimension (actually measured value) of the winding" are shown. As can be seen from the column "diametrical dimension (actually measured value) of the winding" and the column "difference", the diametrical dimension $D(i)$ of the winding obtained using the calculation loop of Eqs. (4) to (6) produces differences of 0.1 to 0.7 mm with the actually measured diametrical dimensions of the winding. Thus, there are almost no errors.

According to the calculational method shown in the present Embodiment 2, the winding diameter assumed after the winding core is rotated through a given rotational angle can be calculated based on the winding diameter computed immediately previously. By implementing this calculational method a required number of times, the winding diameter can be calculated accurately with less error with respect to the actually measured value.

[EMBODIMENT 3]

A method of incrementally correcting the calculated winding diametrical dimension $D(i)$ of the pile beam 20 in Embodiment 2 above is described with reference to Table 3.

In some cases, as the residual thread lengthwise dimension $La(i)$ is reduced, the error between the calculated value of the winding diametrical dimension $D(i)$ and the actually measured value increases. In such cases, during the process in which the warp sheet 12 is being drawn out, the value of the diametrical dimension $D(i)$ of the winding or a value associated with the calculated value of the winding diametrical dimension $D(i)$ is corrected in steps according to the calculated value of the diametrical dimension $D(i)$ of the winding or a value associated with it, to reduce the error between the calculated value and actually measured value of the diametrical dimension $D(i)$ of the winding.

Specifically, the following step 3 -1 is executed after the residual thread lengthwise dimension $Lb(i)$ is calculated using Eq. (5) in step 3 of the above Embodiment 2. Then, the diametrical dimension $D(i)$ of the winding is calculated using Eq. (6) in step 4.

(Step 3 -1) The value of the diametrical dimension $D(i - 1)$ of the winding and the values of the diametrical dimension of the winding in the column "correction time" of Table 3 are compared. A decision is made as to whether it is the time when the value of the residual thread lengthwise dimension $La(i)$ should be corrected. If it is judged that it is the time when the value should be corrected, the value of the residual thread lengthwise dimension $La (i)$ calculated using Eq. (5) is applied to the calculational formula shown in the column "corrective coefficient and calculational formula" of Table 3 according to the value of the diametrical dimension $D(i - 1)$ of the winding, and a correction is made in such a way that the obtained value is taken as the value of the corrected residual thread lengthwise dimension $La(i)$.

<div align="center">Table 3</div>

| $Dmax$ = 459.0 (mm) | | |
|---|---|---|
| Correction time (Winding diametrical dimension $D(i)$ (mm)) | Subject of correction | Corrective coefficient and calculational formula |
| 450 | Residual thread lengthwise dimension La(i) (mm) | $La(i) \times 1.009$ $(La(i) + La(i) \times 0.009)$ |
| 400 | Residual thread lengthwise dimension La(i) (mm) | $La(i) \times 1.009$ $(La(i) + La(i) \times 0.009)$ |
| 350 | Residual thread lengthwise dimension La(i) (mm) | $La(i) \times 1.009$ $(La(i) + La(i) \times 0.009)$ |
| 320 | Residual thread lengthwise dimension La(i) (mm) | $La(i) \times 1.01$ $(La(i) + La(i) \times 0.01)$ |
| 300 | Residual thread lengthwise dimension La(i) (mm) | $La(i) \times 1.01$ $(La(i) + La(i) \times 0.01)$ |
| 280 | Residual thread lengthwise dimension La(i) (mm) | $La(i) \times 1.015$ $(La(i) + La(i) \times 0.015)$ |
| 260 | Residual thread lengthwise dimension La(i) (mm) | $La(i) \times 1.02$ $(La(i) + La(i) \times 0.02)$ |
| 240 | Residual thread lengthwise dimension La(i) (mm) | $La(i) \times 1.04$ $(La(i) + La(i) \times 0.04)$ |

After executing the step 3-1 described above, the corrected residual thread lengthwise dimension $La(i)$ is substituted into Eq. (6) to calculate the winding diametrical dimension $D(i)$ in step 4 of Embodiment 2. Thus, the value of the winding diametrical dimension $D(i)$ of the winding can be corrected.

In table 3, "correction time" means the timing at which a correction is made in step 3-1. In particular, the correction in step 3-1 is performed when the value of the winding diametrical dimension $D(i)$ calculated by the calculation loop every time reaches the value put in the column "correction time". In this way, step 3-1 is not always executed whenever step 3 is carried out. Rather, it is executed whenever either the value of the winding diametrical dimension $D(i)$ or a value regarding the calculated winding diametrical dimension $D(i)$ that gradually varies during the process in which the warp sheet 12 is being drawn out reaches a given value or a given amount of change occurs.

The "subject of correction" in Table 3 means a subject to be corrected. It canbe the residual thread lengthwise dimension $La(i)$. In addition, it can be a value used to calculate the winding diametrical dimension and winding diametrical dimension *D(i)* that varies gradually as the process in which the warp sheet 12 is drawn out, i.e., the calculation loop, is repeated such as the accumulated value of the drawn-out lengthwise dimension *Lb(i)*, the accumulated value of the rotational angle θ*(i)* of the winding core 16, and the residual cross-sectional area *a*. In Table 3, plural "correction times" are set. Only one correction time may be set. Furthermore, the subject of the "correction time" is defined similarly to the "subject of correction".

The corrective coefficient of the "corrective coefficient and calculational formula" in Table 3 is an empirical value previously found by a weaving test. The calculational formula of the "corrective coefficient and calculational formula" is a formula giving a method consisting of multiplying the value of the "subject of correction" by a corrective coefficient, taking the product as an amount of correction, and adding this amount of correction to the value of the subject of correction.

The "correction time", "subject of correction", and "corrective coefficient and calculational formula" of Table 3 are stored in the memory 52 preset by the setting device 50 in Fig. 1.

Where the accumulated value either of the drawn-out lengthwise dimension *Lb(i)* or of the rotational angle θ*(i)* of the winding core 16 is used as the subject of "correction time" or "subject of correction", the arithmetic unit 48 accumulates the value of the drawn-out lengthwise dimension *Lb(i)* or rotational angle θ*(i)* calculated every time.

The step 3-1 of the ith calculation loop for calculating the winding diametrical dimension *D(i)* is described for the case where the value of the column "correction time" of Table 3 is 450 mm.

In the step 3-1 of the ith calculation loop, a decision as to whether the value of the diametrical dimension *D(i)* of the winding is corrected or not is made depending on whether the value of the diametrical dimension *D(i - 1)* of the winding is less than 450 mm in the column "correction time" shown in Table 3 and first preset. Where the value of the diametrical dimension of the winding first satisfies the relation *D(i - 1)* ≦ 450 mm, the residual thread lengthwise dimension *La(i)* is corrected using the calculational formula shown in the column "corrective coefficient and calculational formula" and corresponding to 450 mm in the column "correction time".

Where the ith calculation loop for calculation of the winding diametrical dimension *D(i)* results in "correction time" of 450 mm, if the value of the residual thread lengthwise dimension *La(i)* is corrected based on step 3-1, the corrected value of the residual thread lengthwise dimension *La(i)* is greater than the uncorrected value of the residual thread lengthwise dimension *La(i)*. Therefore, the value of the diametrical dimension *D(i)* of the winding calculated using Eq. (6) and the corrected value of the residual thread lengthwise dimension *La(i)* is greater than the immediately preceding value of the diametrical dimension *D(i - 1)* of the winding.

At this time, the value of the diametrical dimension *D(i)* of the winding may be greater than 450 mm of the "correction time" due to a correction. In this case, in a subsequent calculation loop, a correction corresponding to 450 mm is again made. The value of the winding diametrical dimension *D(i)* varies up and down about 450 mm. This will increase the error between the actually measured value and calculated value of the diametrical dimension *D(i)* of the winding. Accordingly, correction of the residual thread lengthwise dimension *La(i)* corresponding to 450 mm of the "correction time" is carried out only once throughout the whole calculation loop sequence.

In this way, the correction of the residual thread lengthwise dimension *La(i)* corresponding to each "correction time" is made only once. This prevents the value of each correction time from varying up and down about the value of each correction time.

The subject of "correction time" and the "subject of correction" can be combined at will as long as they are values of the winding diametrical dimension or values used for the calculation of the winding diametrical dimension. For example, the subject of "correction time" may be the residual thread lengthwise dimension, and the "subject of correction" may be the diametrical dimension of the winding. The subject of "correction time" may be the residual cross-sectional area of the warp sheet, and the "subj ect of correction" maybe the drawn-out lengthwise dimension.

[OTHER EMBODIMENTS]

If the present invention is applied to towel looms and denim weaving machines, the amount of warp consumed can be managed precisely. The cloth weight can be made uniform among individual sheets. Consequently, the quality of the woven cloth is improved.

The shrinkage rate in the warp direction (ratio of the length of the woven cloth to the unit length of the warp sheet) can be calculated easily and precisely by measuring the length of the woven cloth with the friction roll. Therefore, the dimensions of the completed woven cloth can be graphed precisely. The production rate of off-specification products can be reduced.

The control unit 24 calculates the pile ratio for the pile beam 20 and ground beam 22 from the ratio of the drawn-out lengthwise dimensions calculated by the aforementioned calculational method. Where the difference between each calculated pile ratio and a preset target pile ratio exceeds an allowable range, or where each calculated pile ratio exceeds the allowable range of the preset target pile ratio, the pile tension roll 30 and groundtension roll 32 or terrymotionmechanism 38 may be adjusted by feedback control to achieve the target pile ratio.

In the above Embodiments 1, 2, and 3, the present invention is applied to the warp sheet 12 and winding core 16. The invention can also be applied to the warp sheet 14 and winding core 18.

The present invention is applied to the pile fabric machine 10. Besides, the invention can be applied to other textile machines such as rapier looms and pretreatment machines.

Note that the present invention is not limited to the above-described embodiments. Rather, they can be modified variously as long as the gist is not altered.

According to the method or apparatus for calculating a winding diameter of the present invention, by previously measuring or calculating the diametrical dimension of the winding core, the lengthwise dimension of the sheet-like matter, and the thicknesswise dimension of the sheet-like matter, by entering data about them are entered into an arithmetic unit such as a computer, i.e., the calculational unit, and by making the calculational unit to perform given calculations, the winding diameter can be found without using sensors.

Furthermore, the diametrical dimension $Da$ of the sheet-like matter canbe calculatedbased on the following formula, wherein the lengthwise dimension of the sheet-like matter is $La$, the thicknesswise dimension of the sheet-like matter is $t$, and the diametrical dimension of the winding core is $d$ :

$$Da = \sqrt{\frac{4 \times t \times La}{\pi} + d \times d}$$

The thicknesswise dimension of the sheet-like matter may be previously calculated based on the initial cross-sectional area of the sheet-like matter wound on the winding core and on the total lengthwise dimension of the sheet-like matter within a virtual plane perpendicular to the axis of the winding core.

The cross-sectional area can be calculated based on an actually measured value obtained by actually measuring the initial winding dimension of the sheet-like matter in advance.

The thicknesswise dimension of the sheet-like matter can be calculated based on the following formula, wherein the initial diametrical dimension of the winding formed by the sheet-like matter is $Dmax$, and the initial lengthwise dimension of the sheet-like matter is $Lmax$:

$$t = \frac{\pi \times (D\max \times D\max - d \times d)}{4 \times L\max}$$

Because of the configuration described above, the thicknesswise dimension of the sheet-like matter can be obtained accurately. As a result, the accuracy at which the diametrical dimension of the sheet-like matter that is a winding for which a calculation is to be performed, i.e., the diametrical dimension of the winding, is enhanced.

Furthermore, according to the methods and apparatus for calculating a winding diameter of the present invention, since the winding diameter, when the winding core is rotated through a given rotational angle, the sheet-like matter is drawn out, and the winding diameter decreases gradually, can be calculated, the timing at which the rotational angular speed of the winding core is modified can be set at will.

Furthermore, the methods and apparatus for calculating a winding diameter may further include repetition of the calculation loop in which the calculated winding diametrical dimension is taken as the pre-rotational diametrical dimension. Thus, a gradually decreasing winding diameter can be obtained.

The winding diametrical dimension before the winding core is rotated through the rotational angle can include the initial winding diametrical dimension that is a maximum winding diametrical dimension of the sheet-like matter when it has been wound around the winding core.

When a number indicating the $i$th calculational loop is $i$, the $i$th drawn-out lengthwise dimension is $Lb(i)$, the diametrical dimension of the winding obtained at the $(i - 1)$th time and used as the diametrical dimension of the winding at the $i$th time is $D(i - 1)$, the initial diametrical dimension of the winding formed by the sheet-like matter wound around the winding core within a virtual plane perpendicular to the axis of the winding core is $D(0)$, the ith rotational angle in unit of ° is $\theta(i)$, the $i$th winding diametrical dimension is $D(i)$, the thicknesswise dimension is $t$, the initial lengthwise dimension is $Lmax$, and the $i$th residual thread lengthwise dimension is $La(i)$, the ith drawn-out lengthwise dimension $Lb(i)$, the $i$th residual thread lengthwise dimension $La(i)$, and the $i$th winding diametrical dimension $D(i)$ can be calculated based on the following formulas, respectively.

$$Lb(i) = \frac{\pi \times D(i-1) \times \theta(i)}{360}$$

$$La(i) = L\max - \{Lb(i) + Lb(i-1) + \cdots + Lb(1)\}$$

$$D(i) = \sqrt{\frac{4 \times t \times La(i)}{\pi} + d \times d}$$

Furthermore, in the method and apparatus for calculating a winding diameter, finding the winding diametrical dimension may include calculating the residual cross-sectional area of the sheet-like matter based on the drawn-out lengthwise dimension and on the thicknesswise dimension of the sheet-like matter. Further, the calculation loop may include calculating the winding diametrical dimension indicative of the diametrical dimension of the sheet-like matter when it has been wound around the winding core after the core is rotated through the rotational angle, based on the residual cross-sectional area of the sheet-like matter and on the diametrical dimension of the widing core. According to this method of calculation, it is not necessary to calculate the residual thread lengthwise dimension. Consequently, calculation of the winding diameter can be simplified.

Furthermore, in the method and apparatus for calculation, calculating the diametrical dimension of the winding may include incrementally correcting at least one calculated value selected from a group consisting of the diametrical dimension of the winding, an accumulated value of the drawn-out lengthwise dimension, an accumulated value of the rotational angle, and the residual cross-sectional area according to the calculated value whenever the at least one calculated value reaches a preset given value or whenever a given amount of change occurs.

Furthermore, in the methods and apparatus for calculation, calculating the diametrical dimension of the winding may include incrementally correcting at least one calculated value selected from a group consisting of the diametrical dimension of the winding, an accumulated value of the drawn-out lengthwise dimension, an accumulated value of the rotational angle, the residual cross-sectional area, and the residual thread lengthwise dimension according to the calculated value of the residual thread lengthwise dimension whenever the calculated value of the residual thread lengthwise dimension reaches a preset given value or whenever a given amount of change occurs.

Furthermore, in the methods and apparatus for calculation, calculating the diametrical dimension of the winding may include correcting the diametrical dimension of the winding by incrementally correcting the residual thread lengthwise dimension according to the calculated value of the diametrical dimension of the winding whenever said calculated value of the diametrical dimension of the winding reaches a preset given value or whenever a given amount of change occurs.

## Claims

1. A calculation method, for calculating a diametrical dimension of a winding in a textile machine (10), the winding comprises a sheet-like matter wound around a winding core (16, 18) and including a warp sheet (12, 14) that comprises a plurality of warp threads arranged like a sheet, comprising a step of executing a calculation loop including:

   calculating a drawn-out lengthwise dimension of the sheet-like matter from the winding core when the winding core has been rotated through a rotational angle, based on a diametrical dimension of the winding core, on the rotational angle, and on a pre-rotational diametrical dimension that is a diametrical dimension of a range of the sheet-like matter wound around the winding core before the winding core is rotated through the rotational angle;
   calculating a residual thread lengthwise dimension indicative of a lengthwise dimension of the remaining sheet-like matter, based on an initial lengthwise dimension of the sheet-like matter or a lengthwise dimension of the sheet-like matter before the winding core is rotated through the rotational angle and on the drawn-out lengthwise dimension; and
   calculating a diametrical dimension of the winding indicative of a diametrical dimension of the sheet-like matter wound around the winding core after the winding core has rotated through the rotational angle, based on the residual thread lengthwise dimension, on a thicknesswise dimension of the sheet-like matter, and on the diametrical dimension of the winding core.

2. The calculation method according to claim 1, wherein the calculation loop is repeated while taking the calculated diametrical dimension of the winding as the pre-rotational diametrical dimension of the winding.

3. The calculation method according claim 1 or 2, wherein the diametrical dimension of the winding before the winding core (16, 18) is rotated through the rotational angle includes an initial diametrical dimension of the winding that is a maximum diametrical dimension of the sheet-like matter wound around the winding core.

4. The calculation method according to claim 3, wherein an $i$th drawn-out lengthwise dimension $Lb(i)$ is calculated based on

$$Lb(i) = \frac{\pi \times D(i-1) \times \theta(i)}{360} \quad .$$

where $i$ is a number indicating the $i$th calculation loop, $Lb(i)$ is the $i$th drawn-out lengthwise dimension, $D(i-1)$ is a diametrical dimension of the winding obtained at the $(i-1)$th time and used as the diametrical dimension of the winding at the $i$th time, $D(0)$ is the initial diametrical dimension of the sheet-like matter wound around the winding core within a virtual plane perpendicular to the axis of the winding core, $\theta(i)$ is the $i$th rotational angle in unit of "°", $D(i)$ is the $i$th diametrical dimension of the winding, $t$ is the thicknesswise dimension, $Lmax$ is the initial lengthwise dimension, and $La(i)$ is the $i$th residual thread lengthwise dimension,
wherein the $i$th residual thread lengthwise dimension $La(i)$ is calculated based on

$$La(i) = L\max - \{Lb(i) + Lb(i-1) + \cdots + Lb(1)\} \quad ;$$

and
wherein the $i$th diametrical dimension $D(i)$ of the winding is calculated based on

$$D(i) = \sqrt{\frac{4 \times t \times La(i)}{\pi} + d \times d} \quad .$$

5. The calculation method according to one of claims 1 to 4, further including:

incrementally correcting at least one calculated value selected from a group consisting of the diametrical dimension of the winding, an accumulated value of the drawn-out lengthwise dimension, an accumulated value of the rotational angle, and the residual cross-sectional area, according to the calculated value, whenever the at least one calculated value reaches a preset given value or whenever a given amount of change occurs.

6. The calculation method according to one of claims 1 to 4, further including:

incrementally correcting at least one calculated value selected from a group consisting of the diametrical dimension of the winding, an accumulated value of the drawn-out lengthwise dimension, an accumulated value of the rotational angle, the residual cross-sectional area, and the residual thread lengthwise dimension, according to the accumulated value of the residual thread lengthwise dimension, whenever the calculated value of the residual thread lengthwise dimension reaches a preset given value or whenever a given amount of change occurs.

7. The calculation method according to claim 6, further including:

correcting the diametrical dimension of the winding by incrementally correcting the residual thread lengthwise dimension, according to the calculated value of the diametrical dimension of the winding, whenever the calculated value of the diametrical dimension of the winding reaches a preset given value or whenever a given amount of change occurs.

8. The calculation method according to one of claims 1 to 7, wherein the thicknesswise dimension of the sheet-like matter is previously calculated based on an initial cross-sectional area of the sheet-like matter wound around the winding core (16, 18) within a virtual plane perpendicular to the axis of the winding core, and on a total lengthwise dimension of the sheet-like matter.

9. The calculation method according to claim 8, wherein the cross-sectional area is calculated based on an actually measured value obtained by previously actually measuring an initial diametrical dimension of the sheet-like matter wound around the winding core (16, 18), and wherein the thicknesswise dimension $t$ is calculated based on

$$t = \frac{\pi \times (D\max \times D\max - d \times d)}{4 \times L\max}$$

where $Dmax$ is the initial diametrical dimension of the sheet-like matter wound, and $Lmax$ is the initial lengthwise dimension of the sheet-like matter.

10. An apparatus, for calculating a diametrical dimension of a winding in a textile machine (10), the winding comprises a sheet-like matter wound around a winding core (16, 18) and including a warp sheet (12, 14) that comprises a plurality of warp threads arranged like a sheet, comprising:

   a rotational angle detector for detecting a rotational angle of the winding core when it is rotated;
   a storage portion for storing a diametrical dimension of the winding core, the rotational angle detected by the rotational angle detector, and a pre-rotational diametrical dimension indicative of a diametrical dimension of a range of the sheet-like matter wound around the winding core before the winding core is rotated through the rotational angle; and
   a calculation unit (24) for executing a calculation loop including the steps of calculating a drawn-out lengthwise dimension of the sheet-like matter
   from the winding core when the core is rotated through the rotational angle based on the diametrical dimension of the winding core, the rotational angle, and the pre-rotational diametrical dimension of the winding stored in the storage portion,
   calculating a residual thread lengthwise dimension indicative of a lengthwise dimension of the remaining sheet-like matter based on an initial lengthwise dimension of the sheet-like matter or a lengthwise dimension of the sheet-like matter assumed before the winding core is rotated through the rotational angle and on the drawn-out lengthwise dimension, and
   calculating a diametrical dimension indicative of a diametrical dimension of the sheet-like matter wound around the winding core after the winding core has rotated through the rotational angle based on the residual thread lengthwise dimension, on a thicknesswise dimension of the sheet-like matter, and on the diametrical dimension of the winding core.

**Patentansprüche**

1. Berechnungsverfahren zum Berechnen einer diametralen Abmessung einer Wicklung in einer Textilmaschine (10), wobei die Wicklung ein bahnartiges Material umfasst, das um einen Wicklungskern (16, 18) gewickelt ist und eine Kettfadenbahn (12, 14) umfasst, die eine Vielzahl von Kettfäden umfasst, die wie eine Bahn angeordnet sind, das einen Schritt zum Ausführen einer Berechnungsschleife umfasst, der umfasst:

   Berechnen einer der Länge nach von dem Wicklungskern gezogenen Abmessung des bahnartigen Materials, wenn der Wicklungskern um einen Drehwinkel gedreht wurde, basierend auf einer diametralen Abmessung des Wicklungskerns, auf dem Drehwinkel und auf einer diametralen Abmessung vor dem Drehen, welche eine diametrale Abmessung eines Bereichs des bahnartigen Materials ist, das um den Wicklungskern gewickelt ist, bevor der Wicklungskern um den Drehwinkel gedreht wird;
   Berechnen einer Restfadenabmessung der Länge nach, welche eine Abmessung des restlichen bahnartigen Materials in der Längsrichtung anzeigt, basierend auf einer Anfangsabmessung des bahnartigen Materials in der Längsrichtung oder einer Abmessung des bahnartigen Materials in der Längsrichtung, bevor der Wicklungskern um den Drehwinkel gedreht wird, und auf der in der Längsrichtung ausgezogenen Abmessung; und
   Berechnen einer diametralen Abmessung der Wicklung, die eine diametrale Abmessung des um den Wicklungskern gewickelten bahnartigen Materials anzeigt, nachdem der Wicklungskern um den Drehwinkel gedreht wurde, basierend auf der Abmessung der Restfadenlänge in der Längsrichtung, auf einer Abmessung des bahnartigen Materials in der Dickenrichtung und auf der diametralen Abmessung des Wicklungskerns.

2. Berechnungsverfahren nach Anspruch 1, wobei die Berechnungsschleife wiederholt wird, während die berechnete diametrale Abmessung der Wicklung als die diametrale Abmessung vor dem Drehen der Wicklung genommen wird.

3. Berechnungsverfahren nach Anspruch 1 oder 2, wobei die diametrale Abmessung der Wicklung, bevor der Wicklungskern (16, 18) um den Drehwinkel gedreht wird, eine diametrale Anfangsabmessung der Wicklung umfasst, die eine maximale diametrale Abmessung des bahnartigen Materials ist, das um den Wicklungskern gewickelt ist.

4. Berechnungsverfahren nach Anspruch 3, wobei eine i-te der Länge nach ausgezogene Abmessung Lb(i) berechnet wird basierend auf:

$$Lb(i) = \frac{\pi \times D(i-1) \times \theta(i)}{360}.$$

wobei i eine Zahl ist, die die i-te Berechnungsschleife anzeigt, Lb(i) die i-te der Länge nach ausgezogene Abmessung ist, D(i-1) eine diametrale Abmessung der Wicklung ist, die beim (i-1)-ten Mal erhalten wird und als die diametrale Abmessung der Wicklung beim i-ten Mal verwendet wird, D(0) die diametrale Anfangsabmessung des um den Wicklungskern gewickelten bahnartigen Materials innerhalb einer virtuellen Ebene ist, die senkrecht zu der Achse des Wicklungskerns ist, θ(i) der i-te Drehwinkel in der Einheit "°" ist, D(i) die i-te diametrale Abmessung der Wicklung ist, t die Abmessung in der Dickenrichtung ist, Lmax die Anfangslängsabmessung ist und La(i) die i-te Restfadenlängenabmessung ist,
wobei die i-te Restfadenlängenabmessung La(i) berechnet wird basierend auf:

$$La(i) = Lmax - \{Lb(i) + Lb(i-1) + \ldots + Lb(1)\};$$

und
wobei die i-te diametrale Abmessung D(i) der Wicklung berechnet wird basierend auf:

$$D(i) = \sqrt{\frac{4 \times t \times La(i)}{\pi} + d \times d}.$$

5. Berechnungsverfahren einem der Ansprüche 1 bis 4, das ferner umfasst:

inkrementelles Korrigieren wenigstens eines berechneten Werts, der aus einer Gruppe ausgewählt wird, die besteht aus: der diametralen Abmessung der Wicklung, einem akkumulierten Wert der der Länge nach ausgezogenen Abmessung, einem akkumulierten Wert des Drehwinkels und der Restquerschnittsfläche entsprechend dem berechneten Wert, immer wenn der wenigstens eine berechnete Wert einen vorbestimmten vorgegebenen Wert erreicht oder wenn ein vorgegebener Änderungsbetrag auftritt.

6. Berechnungsverfahren nach einem der Ansprüche 1 bis 4, das ferner umfasst:

inkrementelles Korrigieren wenigstens eines berechneten Werts, der aus einer Gruppe ausgewählt wird, die besteht aus: der diametralen Abmessung der Wicklung, einem akkumulierten Wert der der Länge nach ausgezogenen Abmessung, einem akkumulierten Wert des Drehwinkels, der Restquerschnittsfläche und der Restfadenlängsabmessung entsprechend dem akkumulierten Wert der Restfadenlängsabmessung, immer wenn der berechnete Wert der Restfadenlängsabmessung einen vorbestimmten vorgegebenen Wert erreicht oder wenn ein vorgegebener Änderungsbetrag auftritt.

**7.** Berechnungsverfahren nach Anspruch 6, das ferner umfasst:

Korrigieren der diametralen Abmessung der Wicklung durch inkrementelles Korrigieren der Restfadenlängsabmessung entsprechend des berechneten Werts der diametralen Abmessung der Wicklung, immer wenn der berechnete Wert der diametralen Abmessung der Wicklung einen vorbestimmten vorgegebenen Wert erreicht oder wenn ein vorgegebener Änderungsbetrag auftritt.

**8.** Berechnungsverfahren nach einem der Ansprüche 1 bis 7, wobei die Abmessung (t) in der Dickenrichtung des bahnartigen Materials vorher basierend auf einer Anfangsquerschnittsfläche des um den Wicklungskern (16, 18) gewickelten bahnartigen Materials innerhalb einer virtuellen Ebene senkrecht zu der Achse des Wicklungskerns und auf einer Gesamtlängsabmessung des bahnartigen Materials berechnet wird.

**9.** Berechnungsverfahren nach Anspruch 8, wobei die Querschnittsfläche basierend auf einem tatsächlich gemessenen Wert berechnet wird, der erhalten wird, indem vorher tatsächlich eine diametrale Anfangsabmessung des um den Wicklungskern (16, 18) gewickelten bahnartigen Materials gemessen wird, und wobei die Abmessung (t) in der Dickenrichtung basierend auf

$$t = \frac{\pi \times (D\max \times D\max - d \times d)}{4 \times L\max}$$

berechnet wird, wobei Dmax die diametrale Anfangsabmessung des gewickelten bahnartigen Materials ist und Lmax die Anfangsabmessung in der Längenrichtung des bahnartigen Materials ist.

**10.** Vorrichtung zum Berechnen einer diametralen Abmessung einer Wicklung in einer Textilmaschine (10), wobei die Wicklung ein bahnartiges Material umfasst, das um einen Wicklungskern (16, 18) gewickelt ist und eine Kettfadenlage (12, 14) umfasst, die eine Vielzahl von Kettfäden umfasst, die wie eine Bahn angeordnet sind, die umfasst:

eine Drehwinkelerfassungseinrichtung zum Erfassen eines Drehwinkels des Wicklungskerns, wenn er gedreht wird;
einen Speicherabschnitt zum Speichern einer diametralen Abmessung des Wicklungskerns, des von der Drehwinkelerfassungseinrichtung erfassten Drehwinkels und einer diametralen Abmessung vor dem Drehen, die eine diametrale Abmessung eines Bereichs des um den Wicklungskern gewickelten bahnartigen Materials anzeigt, bevor der Wicklungskern um den Drehwinkel gedreht wird; und
eine Berechnungseinheit (24) zum Ausführen einer Berechnungsschleife, welche die folgenden Schritte umfasst:

Berechnen einer der Länge nach von dem Wicklungskern gezogenen Abmessung des bahnartigen Materials, wenn der Kern um den Drehwinkel gedreht wird, basierend auf der diametralen Abmessung des Wicklungskerns, auf dem Drehwinkel und auf der diametralen Abmessung der Wicklung vor der Drehung, die in dem Speicherabschnitt gespeichert sind,
Berechnen einer Restfadenabmessung der Länge nach, welche eine Abmessung des restlichen bahnartigen Materials in der Längsrichtung anzeigt, basierend auf einer Anfangsabmessung des bahnartigen Materials in der Längsrichtung oder einer Abmessung des bahnartigen Materials in der Längsrichtung, die angenommen wird, bevor der Wicklungskern um den Drehwinkel gedreht wird, und auf der in der Längsrichtung ausgezogenen Abmessung; und
Berechnen einer diametralen Abmessung der Wicklung, die eine diametrale Abmessung des um den Wicklungskern gewickelten bahnartigen Materials anzeigt, nachdem der Wicklungskern um den Drehwinkel gedreht wurde, basierend auf der Abmessung der Restfadenlänge in der Längsrichtung, auf einer Abmessung des bahnartigen Materials in der Dickenrichtung und auf der diametralen Abmessung des Wicklungskerns.

**Revendications**

**1.** Procédé de calcul destiné à calculer les dimensions du diamètre d'un enroulement dans une machine textile (10), l'enroulement comprenant un matériau en forme de nappe enroulé autour d'un noyau d'enroulement (16, 18) et comprenant une nappe de chaîne (12, 14) qui comprend plusieurs fils de chaîne agencés en nappe, lequel procédé comprenant une étape qui consiste à exécuter une boucle de calcul qui comprend :

- le calcul de la dimension de la longueur du matériau en forme de nappe extraite du noyau d'enroulement lorsque le noyau d'enroulement a tourné d'un angle de rotation, sur base de la dimension du diamètre du noyau d'enroulement, de l'angle de rotation, de la dimension du diamètre avant rotation, c'est-à-dire la dimension du diamètre qui correspond à la longueur de matériau en forme de nappe enroulé autour du noyau d'enroulement avant que le noyau d'enroulement ait tourné de l'angle de rotation,
- le calcul de la dimension de la longueur résiduelle de fil qui indique la dimension de la longueur restante du matériau en forme de nappe sur base de la dimension de la longueur initiale du matériau en forme de nappe ou de la dimension de la longueur du matériau en forme de nappe avant que le noyau d'enroulement ait tourné de l'angle de rotation et sur base de la dimension de la longueur extraite et
- le calcul de la dimension du diamètre de l'enroulement; indicatif de la dimension du diamètre du matériau en forme de nappe enroulé autour du noyau d'enroulement après que le noyau d'enroulement a tourné de l'angle de rotation, sur base de la dimension de la longueur résiduelle de fil, de la dimension de l'épaisseur du matériau en forme de nappe et de la dimension du diamètre du noyau d'enroulement.

2. Procédé de calcul selon la revendication 1, dans lequel la boucle de calcul est répétée en prenant comme dimension du diamètre de l'enroulement avant rotation la dimension du diamètre de l'enroulement qui a été calculée.

3. Procédé de calcul selon les revendications 1 ou 2, dans lequel la dimension du diamètre de l'enroulement avant que le noyau d'enroulement (16, 18) ait tourné de l'angle de rotation est la dimension initiale du diamètre de l'enroulement, c'est-à-dire la dimension du diamètre maximum du matériau en forme de nappe enroulé autour du noyau d'enroulement.

4. Procédé de calcul selon la revendication 3, dans lequel la i$^{\text{ème}}$ dimension $Lb(i)$ de la longueur extraite est calculée sur base de :

$$Lb(i) = \frac{\pi \times D(i-1) \times \theta(i)}{360}$$

dans laquelle $i$ est un nombre qui indique la i$^{\text{ème}}$ boucle de calcul, $Lb(i)$ est la i$^{\text{ème}}$ dimension de la longueur extraite, $D(i-1)$ est la $(i-1)^{\text{ème}}$ dimension du diamètre de l'enroulement obtenu, utilisée comme dimension du diamètre de l'enroulement lors de la i$^{\text{ème}}$ itération, $D(0)$ est la dimension du diamètre initial du matériau en forme de nappe enroulé autour du noyau d'enroulement dans un plan virtuel perpendiculaire à l'axe du noyau d'enroulement, $\theta(i)$ est le i$^{\text{ème}}$ angle de rotation en unité de "°", $D(i)$ est la i$^{\text{ème}}$ dimension du diamètre de l'enroulement, $t$ est la dimension initiale de l'épaisseur, Lmax est la dimension de la longueur initiale et $La(i)$ est la i$^{\text{ème}}$ dimension de la longueur résiduelle de fil,
la i$^{\text{ème}}$ dimension de la longueur résiduelle de fil $La(i)$ étant calculée sur base de :

$$La(i) = L\max - \{Lb(i) + Lb(i-1) + \cdots + Lb(1)\}$$

et la i$^{\text{ème}}$ dimension du diamètre $D(i)$ de l'enroulement étant calculée sur base de :

$$D(i) = \sqrt{\frac{4 \times t \times La(i)}{\pi} + d \times d}$$

5. Procédé de calcul selon l'une des revendications 1 à 4, comprenant en outre les étapes qui consistent à :

corriger par incréments au moins une valeur calculée sélectionnée dans l'ensemble constitué de la dimension du diamètre de l'enroulement, de la valeur cumulée de la dimension de la longueur extraite, de la valeur cumulée de l'angle de rotation et de la section transversale résiduelle en fonction de la valeur calculée chaque fois que la ou les valeurs calculées ont atteint une valeur donnée prédéterminée ou chaque fois qu'un niveau donné de modification est survenu.

6. Procédé de calcul selon l'une des revendications 1 à 4, et comprenant en outre l'étape qui consiste à :

corriger par incréments au moins une valeur calculée sélectionnée dans l'ensemble constitué de la dimension du diamètre de l'enroulement, de la valeur cumulée de la dimension de la longueur extraite, de la valeur cumulée de l'angle de rotation, de la section transversale résiduelle et de la dimension de la longueur résiduelle de fil en fonction de la valeur cumulée de la dimension de la longueur résiduelle du fil chaque fois que la valeur calculée de la dimension de la longueur résiduelle du fil a atteint une valeur donnée prédéterminée ou chaque fois qu'un niveau donné de modification est survenu.

7. Procédé de calcul selon la revendication 6, qui comprend en outre l'étape qui consiste à :

corriger la dimension du diamètre de l'enroulement en corrigeant par incréments la dimension de la longueur résiduelle de fil en fonction de la valeur calculée de la dimension du diamètre de l'enroulement chaque fois que la valeur calculée de la dimension du diamètre de l'enroulement a atteint une valeur donnée prédéterminée ou chaque fois qu'un niveau donné de modification est survenu.

8. Procédé de calcul selon l'une des revendications 1 à 7, dans lequel la dimension dans le sens de l'épaisseur du matériau en forme de nappe est calculée préalablement sur base de la section transversale initiale du matériau en forme de nappe enroulé autour du noyau d'enroulement (16, 18) à l'intérieur d'un plan virtuel perpendiculaire à l'axe du noyau d'enroulement et sur base de la dimension de la longueur totale du matériau en forme de nappe.

9. Procédé de calcul selon la revendication 8, dans lequel la superficie de la section transversale est calculée sur base d'une valeur effectivement mesurée obtenue en ayant mesuré effectivement et préalablement la dimension initiale du diamètre du matériau en forme de nappe enroulé autour du noyau d'enroulement (16, 18), la dimension t dans le sens de l'épaisseur étant calculée sur base de :

$$t = \frac{\pi \times (D\max \times D\max - d \times d)}{4 \times L\max}$$

dans laquelle *Dmax* est la dimension initiale du diamètre du matériau en forme de nappe qui a été enroulé et *Lmax* est la dimension de la longueur initiale du matériau en forme de nappe.

10. Appareil de calcul de la dimension du diamètre d'un enroulement dans une machine textile (10), l'enroulement comprenant un matériau en forme de nappe enroulé autour d'un noyau d'enroulement (16, 18) et contenant une nappe de chaîne (12, 14) qui comprend plusieurs fils de chaîne agencés en nappe, et comprenant :

- un détecteur d'angle de rotation qui détecte l'angle de rotation du noyau d'enroulement lorsqu'il est mis en rotation,
- une partie de mémoire qui conserve en mémoire la dimension du diamètre du noyau d'enroulement, l'angle de rotation détecté par le détecteur d'angle de rotation et une dimension du diamètre avant rotation qui indique la dimension du diamètre d'une longueur de matériau en forme de nappe enroulé autour du noyau d'enroulement avant que le noyau d'enroulement ait tourné de l'angle de rotation et
- une unité de calcul (24) qui exécute une boucle de calcul qui comprend les étapes qui consistent à :

calculer la dimension de la longueur extraite du matériau en forme de nappe du noyau d'enroulement lorsque le noyau tourne de l'angle de rotation, sur base de la dimension du diamètre du noyau d'enroulement, de l'angle de rotation et de la dimension du diamètre avant rotation de l'enroulement conservés dans la partie de mémoire,
calculer la dimension de la longueur résiduelle de fil qui indique la dimension de la longueur restante du matériau en forme de nappe sur base de la dimension initiale de la longueur du matériau en forme de nappe ou de la dimension de la longueur du matériau en forme de nappe supposée avant que le noyau d'enroulement ait tourné de l'angle de rotation et sur base de la dimension de la longueur extraite et

calculer la dimension du diamètre qui indique la dimension du diamètre de la nappe autour du noyau d'enroulement après que le noyau d'enroulement a tourné de l'angle de rotation, sur base de la dimension de la longueur résiduelle du fil, de la dimension de l'épaisseur du matériau en forme de nappe et de la dimension du diamètre du noyau d'enroulement.

# Fig.1

EP 1 502 978 B1

# Fig.2

EP 1 502 978 B1

## Fig.3

Actually measured and calculated values of winding diametrical dimension

(mm)

Winding diametrical dimension

Number of woven sheets n

O  Diametrical dimension
   (Calculated value)

●  Diametrical dimension
   .(Actually measued value)

—  Linear Line
   (Diametrical dimension
    (actually measured))

**EP 1 502 978 B1**

## REFERENCES CITED IN THE DESCRIPTION

### Patent documents cited in the description

- JP 4289242 A
- EP 1277682 A